# EUROPEAN PATENT APPLICATION

(11) **EP 1 923 092 A1**
(43) Date of publication of application: **21.05.2008**
(21) Application number: 07120915.9
(22) Date of filing: 16.11.2007
(51) Int. Cl.: A61N 1/08, A61N 1/32, A61N 1/04

(54) **Low-frequency electric therapy apparatus**

(30) Priority: 20.11.2006 JP 2006312480
(71) Applicant: Omron Healthcare Co., Ltd., Kyoto-shi, Kyoto 615-0084 (JP)
(72) Inventor: Miki, Akitoshi, Kyoto 615-0084 (JP); Mizuta, Yoshikazu, Kyoto 615-0084 (JP); Asai, Rika, Kyoto 615-0084 (JP)
(74) Representative: Wilhelms . Kilian & Partner Patentanwälte

(57) **Abstract**

A low-frequency electric therapy apparatus is provided with: a pair of pads 300 and 300 each of which is capable of changing the area of an energizing area; and a wave form generation and output portion 250 which applies a treating current between an energizing area of one pad 300 and that of the other pad 300. A wave form generation and output portion 250 makes a difference in the area of an energizing area between two pads 300 so that a bodily sensation at the pad having a smaller area is more intense than that at the pad having a larger area. Thereby, there may be produced a clear difference in the bodily sensation between the two pads.

## Description

### BACKGROUND OF THE INVENTION

### Field of the Invention

The present invention relates to a low-frequency electric therapy apparatus.

### Description of the Related Art

There has been a low-frequency electric therapy apparatus, by which a treating current of a low frequency is energized between electrode pads applied to a body for treatment. It has been known that, in this kind of a low-frequency electric therapy apparatus, a more intense bodily sensation is obtained not at a pad on the positive electrode side than at a pad on the negative electrode side (that is, at the pad into which a current flows). There has been a conventional product by which, using the above characteristic, different kinds of bodily sensations from each other in the intensity are obtained at the both right and left pads.

However, a difference in the polarities has caused a not-so-big difference in the bodily sensations, and there has been caused no clear difference in the bodily sensation between a right and left pads.

Here, in Japanese Patent No. 2917270 (Japanese Patent Application Laid-Open No. 09-38215), there has been proposed a low-frequency electric therapy apparatus which has a single pad provided with three or more electrodes, and may change the number of positive electrodes and that of negative electrodes by switching the polarities of the electrodes.

### SUMMARY OF THE INVENTION

The present invention has been made, considering the above circumstance, and its object is to provide a low-frequency electric therapy apparatus which produces a clear difference of the bodily sensation between the two pads.

In order to realize the above-described object, the low-frequency electric therapy apparatus according to the present invention has adopted the following configuration.

The low-frequency electric therapy apparatus according to the present invention is provided with: a pair of pads each of which is capable of changing the area of the energizing area; and an output portion which applies a treating current between an energizing area of one pad and that of the other pad, wherein the above-described output portion makes a difference in the area of the energizing area between two pads to realize so that a bodily sensation at a pad having a smaller area is more intense than that at a pad having a larger area. Here, "energizing area" means the whole area or a part of the whole area of the pad, and a range within which a treating current flows.

According to the above configuration, amore intense bodily sensation is obtained at a pad having the smaller area than that of a pad having the larger area because currents concentrate in a pad having the smaller area. Accordingly, it is possible to produce a clearer difference in the bodily sensation between the two pads in comparison with that of a conventional case.

It is preferable to adopt a configuration in which the above-described output portion applies a treating current in a state that the above-described pad having a larger area is a positive electrode, and the above-described pad having a smaller area is a negative electrode. There is a larger difference in the bodily sensation because there is a characteristic that the bodily sensation is more intense at the negative electrode side in comparison with that at the positive electrode side.

Preferably, the above-described pad has a plurality of electrodes each of which may individually switch the conducting and the non-conducting state, and changes the area of the energizing area by changing the number of conducting electrodes. According to the above configuration, it is simple to realize a change in the area of the energizing area.

It is preferable for the above-described output portion to move the position of the energizing area in the pad having the above-described smaller area.
Thereby, the position of the intense bodily sensation (energizing area in the pad having smaller area) may be moved in the pad.

It is preferable for the above-described output portion to automatically move the position of the energizing area of a pad having the above-described smaller area while a treating current is being output. For example, when the energizing area is moved while a treating current corresponding to "tapping" and "pushing" is output, a point at which "tapping" and "pushing" are performed with a more intense bodily sensation is moved in the pad to produce a bodily sensation very similar to massaging performed by a person.

The low-frequency electric therapy apparatus is preferably provided with a plurality of switches corresponding to each electrode of each pad, and, when the above-described switch is pushed, the above-described output portion puts an electrode corresponding to the pushed switch into a conducting state, other electrodes in the same pad into a cutting state, and, at the same time, two or more electrodes in the other pad into a conducting state. The operability and the convenience of the low-frequency electric therapy apparatus may be improved because the bodily sensation may be adjusted by an easy and intuitive operation of pushing a switch corresponding to an electrode at a position at which a bodily sensation is required to be intensified.

Preferably, the low-frequency electric therapy apparatus is further provided with a display portion displaying energizing areas in each pad, and the above-described display portion emphatically displays the energizing area in a pad having a smaller area. By seeing the above display, a user may easily confirm a position at which a bodily sensation is intense. Moreover, a user may confirm the result of operation performed by the user himself or herself because the above display is performed in synchronization with the operation of the above-described switch.

The present invention may be understood as a low-frequency electric therapy apparatus having at least a portion of the above-described means, as a method for controlling a low-frequency electric therapy apparatus including at least a part of the above-described processing, as a program realizing the above method, or as a recording medium recording the above program. Here, each of the above-described means and processing may be combined each other to form the present invention.

According to the present invention, it is possible to produce a clearer difference in the bodily sensation between the two pads in a low-frequency electric therapy apparatus provided with a pair of pads, and to have hitherto unknown new massaging effects.

### BRIEF DESCRIPTION OF THE DRAWINGS

FIG. 1 is a drawing showing an external view of a low-frequency electric therapy apparatus;
FIG. 2 is a drawing showing an external view of an operation portion;
FIG. 3 is a block diagram schematically showing a configuration of the low-frequency electric therapy apparatus;
FIG. 4 is a drawing showing one example of treatment wave forms using pulse currents;
FIG. 5A is a drawing showing an energizing area in a standard state;
FIG. 5B is a drawing showing a display example of a display portion corresponding to FIG. 5A;
FIG. 5C is a drawing showing an energizing area when an upper right electrode switch is pushed;
FIG. 5D is a drawing showing a display example of a display portion corresponding to FIG. 5C;
FIG. 6 is a drawing showing one example of the energizing pattern of "tapping"; and
FIG. 7 is a drawing showing one example of the energizing pattern of "pushing".

### DESCRIPTION OF THE EMBODIMENTS

Hereinafter, preferable embodiments according to the present invention will be illustratively explained in detail, referring to the drawings.

### <External View of Low-frequency electric therapy apparatus >

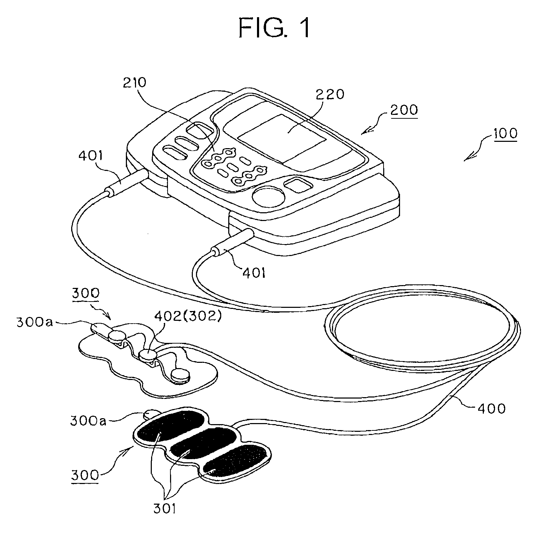

A low-frequency electric therapy apparatus according to an embodiment of the present invention will be explained, referring to FIG. 1. FIG. 1 is a drawing showing an external view of the low-frequency electric therapy apparatus.

A low-frequency electric therapy apparatus 100 roughly includes: a main body 200 of the therapy apparatus; a pair of pads 300 for being applied to a treatment portion; and a cord 400 electrically connecting a main body 200 of the therapy apparatus and the pads 300.

The pad 300 is made of a thin and flexible material. A plurality of electrodes 301 are formed on one surface (a surface contacted with the body) of the pad 300. In the present embodiment, three electrodes 301 are arranged in the longitudinal direction of the pad 300. A convex holding portion 300a is provided on one end portion in the longitudinal direction of the pad 300. This holding portion 300a functions as a mark for determining which side of the pad 300 is the upper side.

The surface of the electrode side of the pad 300 is coated with an adhesive material in a gel state. The film of the gel material is uniformly formed over the whole surface of the pad. The electrodes are not especially electrically insulated from each other, because of the film of the gel material therebetween.

A snap 302 corresponding to each of the electrodes 301 is provided on the other surface of the pad 300. The main body 200 and the pad 300 (electrodes 301) are connected to each other by snap connection between a snap 402 of the cord 400 and the snap 302 on the side of the pad 300 and by inserting a plug 401 of the cord 400 into a jack in the main body 200.

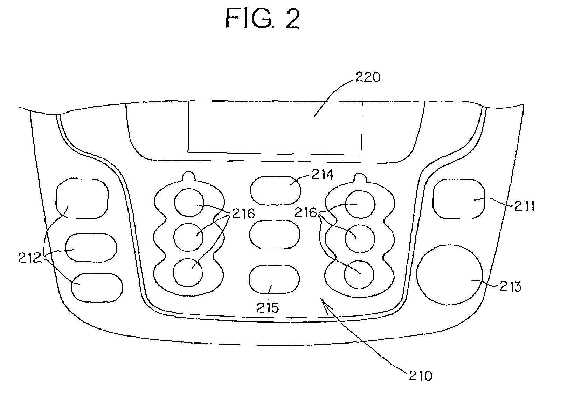

An operation portion 210 including switches and dials, and a display portion 220 including a liquid crystal display (LCD) are provided on the main body 200. As shown in FIG. 2, there are provided on the operation portion 210: a power supply switch 211 for switching on and off of the power supply; a mode selection switch 212 for selecting a treatment mode; an intensity adjustment dial 213 for adjusting the intensity; a position change switch 214 for moving an energizing area; a size change switch 215 for changing the size (area) of the energizing area; an electrode switch 216 for selecting an electrode 301 in an individual manner. A current operation status, a navigating state for operations and pad applying, and messages are displayed in the LCD on the display portion 220.

### <Internal Configuration of Low-frequency electric therapy apparatus>

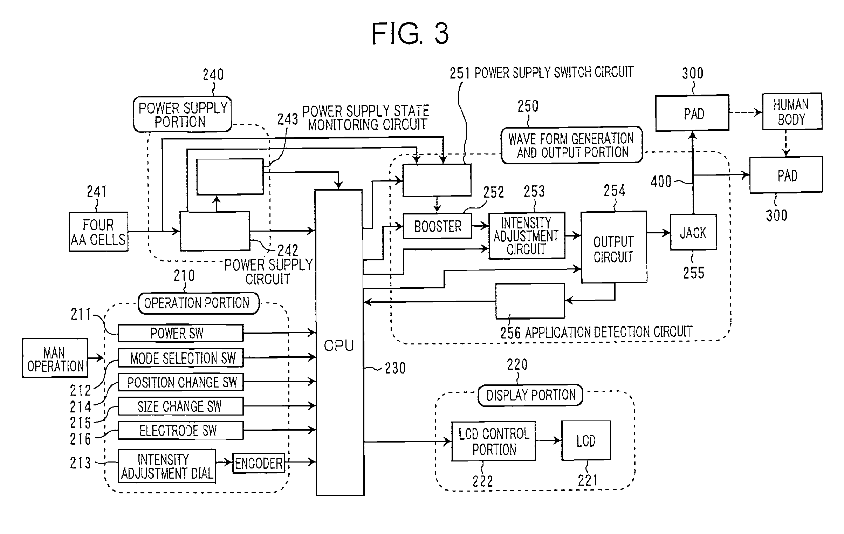

FIG. 3 is a schematic block diagram showing a hardware configuration of the low-frequency electric therapy apparatus. As shown in FIG. 3, the main body 200 roughly includes: an operation portion 210; a display portion 220; a CPU (control portion) 230; a power supply portion 240; and a wave form generation and output portion 250.

As described above, the operation portion 210 includes various kinds of switches and dials. When a user operates a switch or a dial, a corresponding signal is input to the CPU 230. Moreover, an LCD 221, and an LCD control portion 222 controlling the LCD 221 according to a signal from the CPU 230 are provided in the display portion 220.

A power supply circuit 242 and a power supply state monitoring circuit 243 are provided in a power supply portion 240. The low-frequency electric therapy apparatus uses four AA alkaline dry batteries as a power supply 241. As the voltages of batteries may be greatly changed (about 7 V through 3.5 V) according to the consumption of the batteries, the power supply circuit 242 generates a drive voltage (DC 3.3 V) to be supplied to the CPU 230 by voltage down and stabilization of the voltages of the batteries. A DC output from the power supply circuit 242 is supplied to the CPU 230, and, at the same time, is also input to the power supply state monitoring circuit 243. The power supply state monitoring circuit 243 monitors an output voltage from the power supply circuit 242, and stops the CPU 230 (main body 200) when the output voltage becomes lower than 3.3 V.

The wave form generation and output portion 250 is a functional block for increasing an voltage of the power-supply voltage, and for generating and outputting a treating current (treatment wave form), and includes: a power supply switch circuit 251; a booster circuit 252; a intensity adjustment circuit 253; an output circuit 254; a jack 255; an application detection circuit 256; and the like. The power supply switch circuit 251 is a circuit for switching the power-supply voltage supplied to the booster circuit 252, and selects a voltage from the power supply (battery) 241 while treating, and a voltage of 3.3 V from the power supply circuit 242 except for treating. The booster circuit 252 is a circuit boosting a power-supply voltage to a predetermined voltage. The intensity adjustment circuit 253 is a circuit adjusting a voltage boosted in the booster circuit 252 to a voltage corresponding to a set intensity. In the present embodiment, the intensity adjustment may be performed in 21 steps of 0 - 20 levels, using the intensity adjustment dial 213, and an intensity step indicating value set by the dial is input to the intensity adjustment circuit 253 through the CPU 230. The output circuit 254 generates a wave form corresponding to a treatment mode, based on the voltage adjusted in the intensity adjustment circuit 253, and the treatment wave form is output to the electrode 301 of the pad 300 through the jack 255. When operations such as switching of the treatment mode, changing of the energizing area, selection of an electrode, and the like are performed in the operation portion 210, a control signal corresponding to the operation content is input from the CPU 230 to the output circuit 254. The output circuit 254 performs generation of a treatment wave form, switching of a polarity, and switching conducting/non-conducting of each electrode according to the control signal.

The application detection circuit 256 is a circuit which determines whether the pad 300 is correctly applied to the human body by detecting whether an electric current is flowing between the pair of pads 300. The above circuit uses a principle that an electric current does not flow when the pad 300 is not correctly applied, because there is no current loop in which an electric current is output from one of the pads 300, passes through the human body, and returns to the other pad 300.

### <Operation at Treating>

The operation method and the treatment operation of a low-frequency electric therapy apparatus 100 will be explained.

A user is able to select a treatment mode by operating the operation portion 210 (mode selection switch 212). A treatment method such as "kneading", "tapping", "pushing", and "rubbing", a treatment portion such as "shoulder", "arm", and "waist", and the like may be specified as a treatment mode. An "automatic" mode in which a treatment method has been programmed beforehand may be also selected. Moreover, a user may also adjust the intensity by operating the dial.

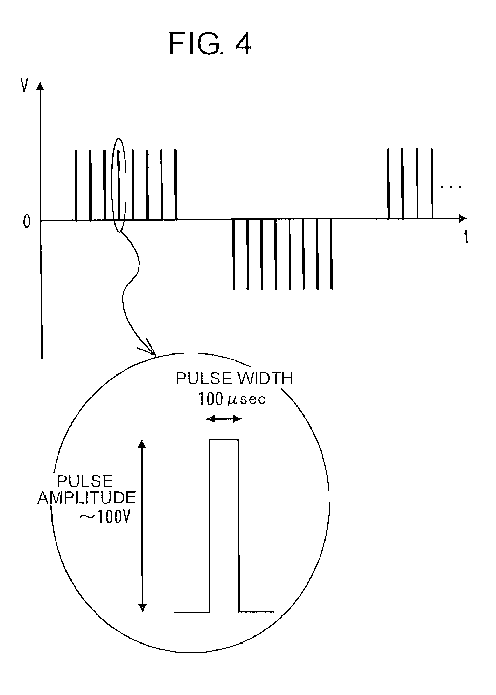

The output circuit 254 generates a treating current of low frequency according to a control signal from the CPU 230. FIG. 4 is a drawing showing one example of treatment wave forms using pulse currents. A pulse with an extremely short width of about 100 microseconds is used as a pulse. The maximum value of the pulse amplitude is about 100 V. A various kinds of stimuli such as "kneading", "tapping", "pushing", and "rubbing" may be produced by changing the wave forms (including polarities, intervals, and arrangements) of the pulses and the like. Moreover, the "intensity" adjustment may be realized by changing the pulse amplitude and the pulse width, and the "speed" adjustment may be realized by changing the occurrence cycle of the pulse group. As a conventional method may be used for specific wave forms, detailed explanation will be omitted. Here, it is acceptable to use an alternating current, instead of a pulse current, as a treating current.

The treating current is output to the electrode 301 of the pad 300 on the higher potential side (when a plurality of electrodes are in a conducting state, an electric current in one system is distributed to the plurality of conducting electrodes). Then, an electric current flows from the electrode (positive electrode) 301 of the pad 300 on the higher potential side to the electrode (negative electrode) 301 of the pad 300 on the low potential side through the human body. A muscle receives an electric stimulus by the treating current flowing between the above pads, and repeats contraction and relaxation to obtain a similar treating effect to that of the massaging.

### <Pinpoint Emphasis of Bodily Sensation>

A low-frequency electric therapy apparatus 100 according to the present embodiment has a function by which only a bodily sensation at a part of one pad 300 is pin-pointedly emphasized. As described hereinafter, there are a case in which a point at which a bodily sensation is intensified is manually specified, and a case in which a CPU 230 automatically selects a point which the bodily sensation is intensified according to the program.

### (Case of Manual Specification)

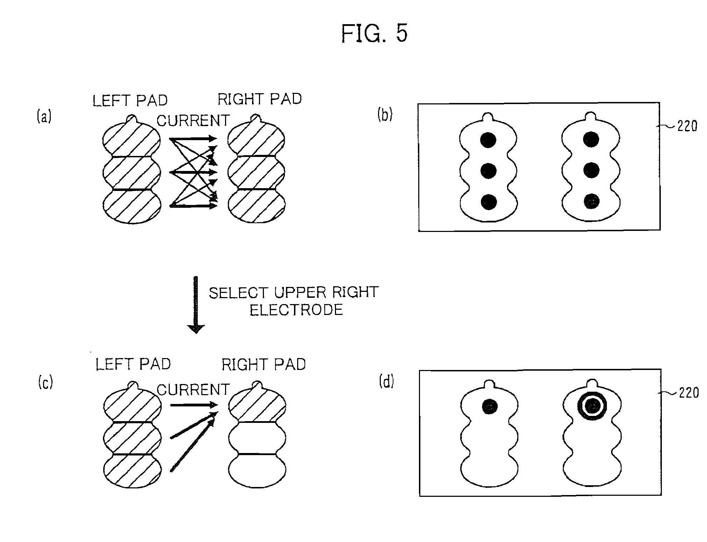

At the starting point (standard state) of treatment operation, all of six electrodes 301 are in the conducting state, and, as shown in FIG. 5A, all surfaces of the pad 300 are an energizing area (hatched portions). FIG. 5B shows a display example of a display portion 220 at the standard state. Frames showing an external shape of the pad 300 are drawn on the display portion 220, and a mark corresponding to an electrode in the conducting state is put into a lighting state. (Hereinafter, for convenience in explaining, three electrodes 301 of the pad 300 are called "upper electrode", "intermediate electrode", and "lower electrode", corresponding to display on the display portion 220.)

As shown in FIG. 2, six electrode switches 216 corresponding to electrodes 301 of each of the right and the left pads 300 are provided in the operation portion 210 of the low-frequency electric therapy apparatus 100. A user may specify a point at which the bodily sensation is required to be intensified, using one of the electrode switches 216.

When the electrode switch 216 is pushed, the output circuit 254 puts the electrode corresponding to the pushed electrode switch 216 into a conducting state, and other electrodes on the same pad into a cut off state, and, at the same time, puts two or more electrodes on the other pad into a conducting state. Thereby, there is caused a difference in the area of the energizing area between the right and left pads. As a current concentrates on one electrode on the pad having the smaller area, the bodily sensation becomes more intense than that of the pad having a larger area. The low-frequency electric therapy apparatus 100 according to the present embodiment realizes pinpoint emphasis of the bodily sensation, using the above principle.

For example, when the upper right electrode switch 216 is pushed, an upper electrode on the right pad and all the electrodes on the left pad are put into a conducting state, as shown in FIG. 5C. Thereby, a bodily sensation at the upper electrode on the right pad is emphasized. Here, the reason why all the electrodes on the left pad which is on a non-emphasis side are put into a conducting state is that the bigger area difference (area ratio) between the right and left pads causes the difference in the bodily sensation to become larger.

Moreover, when the difference in the bodily sensation is required to be larger, it is preferable that the output circuit 254 applies the treating current under a state in which a pad having a larger area is a positive electrode, and a pad having a smaller area is a negative electrode. In the case of FIG. 5C, the treating current is configured to flow from all the electrodes on the left pad into the upper electrode of the right pad. Generally, the bodily sensation is more intense at the negative electrode side in comparison with that at the positive electrode side. Accordingly, there is obtained a clearer difference of the bodily sensation between the right and left pads because a difference of the bodily sensation by the polarities is added to that by the area differences.

FIG. 5D shows a display example corresponding to FIG. 5C. At pinpoint emphasis, there are put into a lighting state only two marks, that is, a mark corresponding to an electrode (the upper electrode on the right pad) at which a bodily sensation is intensified, and a mark corresponding to an electrode (the upper electrode on the left pad), both of which form a pair, and marks corresponding to other electrodes are turned off. Then, a mark corresponding to the electrode (the upper electrode on the right pad) at which a bodily sensation is intensified is displayed in a larger size. By seeing such an emphasized display, a user may easily confirm a position at which the bodily sensation is intensified. Moreover, as a display is made in synchronization with the electrode switch 216, a user may confirm a result obtained by the operation of the user himself or herself. Here, in order to realize an emphasized display, there may be used various kinds of methods such as a blinking display, a colored display, and an animation display, in addition to a method for changing the size and the shape of a mark as shown in FIG. 5C.

As described above, in the low-frequency electric therapy apparatus 100 according to the present embodiment, a bodily sensation may be adjusted by a simple and intuitive operation of pushing the electrode switch 216 corresponding to an electrode at a position at which the bodily sensation is required to be intensified. Accordingly, there may be realized a low-frequency electric therapy apparatus having excellent operability and convenience.

### (Case of Automatic Selection)

In an automatic mode, the CPU 230 controls the area and the position of the energizing area according to a program set beforehand. Hereinafter, there will be explained one example of an energizing pattern (a method changing an energizing area) for each of "tapping" and "pushing". Here, even in the automatic mode, there is performed emphasized display of a mark corresponding to an electrode, at which a bodily sensation is intensified, in the same manner as that of manual specification.

### (1) Energizing Pattern of "Tapping"

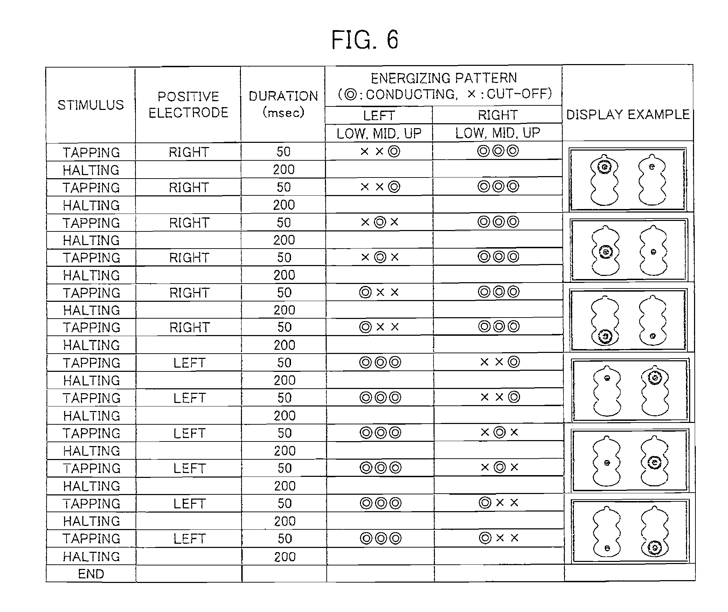

FIG. 6 shows examples of energizing patterns for "tapping". In the table, "halting" shows a state in which a treating current is not output. A treating current with a wave form of tapping flows between the right and left pads during "tapping".
The "left" or the "right" in a column of "positive electrode" means a high potential side (positive electrode side). "Left" means that a current flows from the left pad to the right pad, and "right" means that a current flows from the right pad to the left pad. The symbol 1 of "⊚ (conducting )" and that of "× (cut off)" mean a state of each electrode, and these symbols correspond to, from the left sequentially, the states of the lower electrode, the intermediate electrode, and the upper electrode of the left pad, and the lower electrode, the intermediate electrode, and the upper electrode of the right pad. Display examples of the display portion 220 are also shown in the right side of the table.

When the treatment operation starts, all the electrodes on the right pad, and only the upper electrode on the left pad are put into a conducting state, and a treating current with a wave form of tapping flows from all the electrodes on the right pad to the upper electrode on the left pad for 50 msec. The above operation is corresponding to one time of "tapping". While there is a "tapping" stimulus at each of the right and left pads, a bodily sensation is further intensified at the left pad which has a smaller energizing area and is the negative electrode. That is, a user feels a more intense tapping stimulus at the position of the upper electrode of the leftpad. Amore intense tapping stimulus is output again at the position of the upper electrode on the left pad followed by halting of 200 msec.

Subsequently, the conducting electrode of the left pad is switched to the intermediate electrode, and "tapping" is executed twice. In this case, a user feels a more intense tapping stimulus at the position of the intermediate electrode of the left pad. Then, the conducting electrode of the left pad is switched to the lower electrode, and "tapping" is executed twice. In this case, a user feels a more intense tapping stimulus at a position of the lower electrode of the left pad. As described above, a point at which "tapping" with more intense bodily sensation is performed is moved in the pad by sequentially moving the position of the energizing area of the left pad while a treating current for tapping is output, and a bodily sensation very similar to massaging performed by a person may be produced.

After the pinpoint emphasis of the left pad is executed, the right and left massaging change places, and the pinpoint emphasis of the right pad is executed. That is, all the electrodes of the left pad and only the upper electrode of the right pad first enter into a conducting state, and a treating current for "tapping" is output twice under a state in which the left pad is positive electrode. Subsequently, the conducting electrode of the right pad is switched to the intermediate electrode to execute "tapping" twice, and the conducting electrode is switched to the lower electrode to execute "tapping" twice. As described above, a bodily sensation that massaging is alternately performed may be given to a user by periodically switching a pad performing the pinpoint emphasis.

### (2) Energizing Pattern for "Pushing"

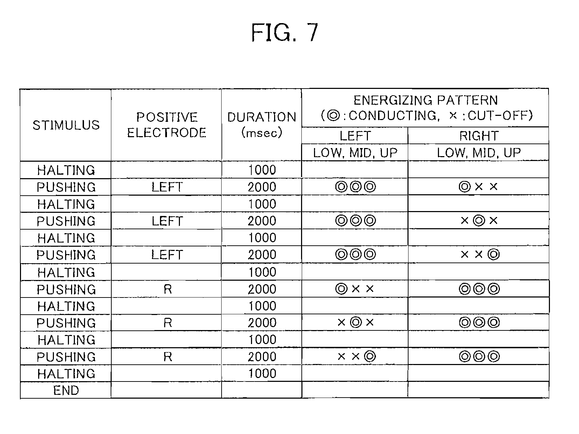

FIG. 7 shows examples of the energizing pattern for "pushing".

When the treatment operation starts, after halting if 1000 msec, all electrodes of the left pad and only the upper electrode of the right pad enter into a conducting state, and a treating current with a pushing wave form flows from all the electrodes of the left pad to the upper electrode of the right pad for 2000 msec. The above operation corresponds to one time of "pushing". While a "pushing" stimulus is also given to each of the right and left pads in this case, a bodily sensation is further strengthened at the right pad which has the smaller energizing area, and is on the negative electrode side. Subsequently, the conducting electrode of the right pad is switched to the intermediate electrode, and, then, to the lower electrode, while placing the resting of 1000 msec, to execute "pushing". As described above, the point at which "pushing" is executed with more intense bodily sensation is moved in the pad by sequentially moving the position of the energizing area of the right pad while outputting the treating current for pushing to produce a bodily sensation very similar to massaging performed by a person.

After pinpoint emphasis of a right pad is executed, the right pad and the left pad change place to execute pinpoint emphasis of the left pad. Thereby, the bodily sensation that more intense massaging of "pushing" is alternately performed may be given to a user.

As described above, the low-frequency electric therapy apparatus 100 according to the present embodiment may produce a clear difference in the bodily sensation between the right pad and the left pad by making the area of one pad smaller and by concentrating the treating current thereon. Moreover, the difference of the bodily sensation between the pads is made clearer by flowing a treating current under a state in which the pad having a smaller area is treated as a negative electrode. Accordingly, a new massaging effect which has not been realized by a conventional low-frequency electric therapy apparatus may be produced.

Here, the above-described embodiment only shows one specific example of the present invention. The scope of the present invention is not limited to the above-described embodiment, and many kinds of variations may be realized within a scope of the technological idea of the present invention.

For example, the number of the pad electrodes is not limited to three. It is acceptable to use one, two, or more than three for the numbers. The shape of the electrode and how to divide the electrode are arbitrary. It is acceptable to two-dimensionally change the position and the size of the energizing area by division in the transverse direction as well as in the longitudinal direction.

## Claims

1. A low-frequency electric therapy apparatus, comprising:
a pair of pads each of which is capable of changing the area of an energizing area; and
an output portion which applies a treating current between an energizing area of one pad and that of the other pad, wherein
said output portion makes a difference in the area of the energizing area between the two pads so that a bodily sensation at the pad having a smaller area is more intense than that at the pad having a larger area.

2. A low-frequency electric therapy apparatus as claimed in claim 1, wherein
said output portion applies a treating current under a state in which said pad having a larger area is a positive electrode, and saidpad having a smaller area is a negative electrode.

3. A low-frequency electric therapy apparatus as claimed in claim 1 or 2, wherein
said pad has a plurality of electrodes each of which may individually switch the conducting and the non-conducting state, and
said output portion changes the area of an energizing area by changing the number of conducting electrodes.

4. A low-frequency electric therapy apparatus as claimed in any one of claims 1 through 3, wherein
said output portion moves the position of an energizing area in said pad having a smaller area.

5. A low-frequency electric therapy apparatus as claimed in claim 4, wherein
said output portion automatically moves the position of an energizing area on said pad having a smaller area while outputting a treating current.

6. A low-frequency electric therapy apparatus as claimed in any one of claims 3 through 5, provided with:
a plurality of switches corresponding to electrodes of each pad; wherein
when said switch is pushed, said output portion puts an electrode corresponding to said pushed switch into a conducting state, the other electrodes on the same pad into a cut off state, and, at the same time, two or more electrodes on the other pad into a conducting state.

7. A low-frequency electric therapy apparatus as claimed in any one of claims 1 through 6, further provided with:
a display portion displaying energizing areas in each of pads, wherein
said display portion emphatically displays an energizing area on a pad having a smaller area.
